(19) Europäisches Patentamt
European Patent Office
Office européen des brevets

(11) Veröffentlichungsnummer: **0 069 754**
**B1**

(12) **EUROPÄISCHE PATENTSCHRIFT**

(45) Veröffentlichungstag der Patentschrift: **10.10.84**

(21) Anmeldenummer: **82900306.0**

(22) Anmeldetag: **21.01.82**

(86) Internationale Anmeldenummer: **PCT/EP 82/00010**

(87) Internationale Veröffentlichungsnummer: **WO 82/02552 (05.08.82** Gazette **82/19)**

(51) Int. Cl.³: **C 07 D 233/60,** C 07 D 233/91, C 07 D 233/68, C 07 D 233/70, C 07 D 409/12, C 07 D 409/06, C 07 D 401/12, C 07 D 401/06

(54) **VERFAHREN ZUR HERSTELLUNG VON IMIDAZOLYLVINYLETHERN.**

(30) Priorität: **23.01.81 CH 430/81**

(43) Veröffentlichungstag der Anmeldung: **19.01.83 Patentblatt 83/3**

(45) Bekanntmachung des Hinweises auf die Patenterteilung: **10.10.84 Patentblatt 84/41**

(84) Benannte Vertragsstaaten: **AT BE DE FR GB NL SE**

(56) Entgegenhaltungen: **EP - A - 0 008 804**

(73) Patentinhaber: **SIEGFRIED AKTIENGESELLSCHAFT, CH-4800 Zofingen (CH)**

(72) Erfinder: **Zirngibl, Ludwig, Dr., Eisengrubenweg 16, CH-4800 Zofingen (CH)**
Erfinder: **THIELE, Kurt, Dr., Rebbergstrasse 47 d, CH-4800 Zofingen (CH)**

(74) Vertreter: **Jaeger, Klaus, Dr. et al, JAEGER & PARTNER Patentanwälte Bergstrasse 48 1/2, D-8035 München-Gauting (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 069 754 B1

**Beschreibung**

Die Erfindung betrifft ein Verfahren zur Herstellung von Imidazolylvinylethern der allgemeinen Formel I

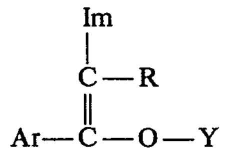

(I)

und deren Säureadditionssalze, wobei

Ar Phenyl, Naphthyl, Thienyl oder Pyridyl bedeutet, wobei diese Ringe einfach oder mehrfach substituiert sein können und die Substituenten unabhängig voneinander Halogen, niederes Alkyl oder Cycloalkyl mit bis zu 6 Kohlenstoffatomen, Trifluormethyl, niederes Alkoxy und niederes Alkylthio mit jeweils 1 bis 6 Kohlenstoffatomen im Alkylteil, Phenyl, Benzyl, Cyano, Nitro oder Amino sein können,

R Wasserstoff, ein unverzweigtes oder verzweigtes Alkyl mit bis zu 6 Kohlenstoffatomen, Phenyl oder ein phenylsubstituiertes Alkyl mit bis zu 6 Kohlenstoffatomen im Alkylteil sein kann, wobei das Phenyl jeweils mit Halogen oder Alkyl oder Alkoxy mit bis zu 6 Kohlenstoffatomen im Alkylteil substituiert sein kann,

Im die 1-H-Imidazol-1-yl-gruppe bedeutet, die einfach mit der Nitrogruppe oder einfach, zweifach oder dreifach mit Halogen oder einem Alkyl- oder Alkoxyrest mit 1 bis 4 Alkylkohlenstoffatomen substituiert sein kann, und

Y ein unverzweigtes, verzweigtes oder cyclisches, gesättigtes oder ungesättigtes, eine Doppelbindung aufweisendes Alkyl mit bis zu 6 Kohlenstoffatomen, Phenyl, Naphthyl, Thienyl oder Pyridyl oder ein mit Phenyl, Naphthyl, Pyridyl oder Thienyl substituiertes Alkyl mit 1 bis 6 Kohlenstoffatomen im Alkylteil bedeutet, wobei alle vorstehend genannten Reste, und zwar auch die Alkylreste, jeweils in der vorstehend für den Rest Ar genannten Weise substituiert sein können und eine direkt an den Ethersauerstoff gebundene Alkylgruppe oder eine direkt an den Ethensauerstoff gebundene Alkylengruppe einmal oder zweimal in der Kette die zweiwertigen Gruppen $-O-$, $-S-$, $-SO-$ oder $-SO_2-$ aufweisen kann oder eine dieser Gruppe in $\omega$-Stellung zum Ethersauerstoff endständig tragen kann, wobei diese Gruppe dann mit einem der für Ar oben genannten Reste abgesättigt ist,

durch Umsetzen eines entsprechenden Ketons der allgemeinen Formel II

$$AR-CO-CHR-Im \qquad (II)$$

in der Ar, R und Im die vorstehend genannte Bedeutung haben, in einem alkalischen Medium mit einer Verbindung der allgemeinen Formel III

$$Z-Y \qquad (III)$$

in der Z eine alkalisch abspaltbare Gruppe ist, und Abtrennen des Reaktionsproduktes.

In der allgemeinen Formel I ist der Rest Ar, also Phenyl, Naphthyl, Thienyl oder Pyridyl, vorzugsweise einfach oder zweifach substituiert, und zwar vorzugsweise mit Halogen, insbesondere Chlor oder Brom, sowie unverzweigtem oder verzweigtem nichtcyclischem Alkyl mit 1 bis 4 Kohlenstoffatomen, vor allem jedoch mit Methyl oder Ethyl. Bei den eingliedrigen Ringsystemen, die der Rest Ar bedeuten kann, stehen die gegebenenfalls vorhandenen Substituenten vorzugsweise in 2- und/oder 4-Stellung.

Der Rest R in der allgemeinen Formel I ist vorzugsweise Wasserstoff oder ein unverzweigtes oder verzweigtes Alkyl mit 1 bis 4 Kohlenstoffatomen, insbesondere Methyl oder Ethyl.

Der Rest Im in der allgemeinen Formel I ist vorzugsweise die unsubstituierte 1-H-Imidazol-1-yl-gruppe oder ist diese Gruppe, die einfach, zweifach oder dreifach, vorzugsweise jedoch einfach, mit Halogen, insbesondere Chlor oder Brom, und/oder Methyl substituiert ist.

Schließlich ist in der allgemeinen Formel I der Rest Y vorzugsweise eine unverzweigte oder verzweigte nichtcyclische gesättigte Alkylgruppe mit 1 bis 6 Kohlenstoffatomen, eine Niederalkoxy-Niederalkylgruppe, eine Phenyl-Niederalkoxy-Niederalkylgruppe, eine Phenoxy-Niederalkylgruppe, eine Phenoxy-Niederalkoxy-Niederalkylgruppe oder eine Niederalkyl-Amino-Niederalkylgruppe, wobei »Niederalkyl« und »Niederalkoxy« in allen vorgenannten Fällen als Gruppen mit 1 bis 4 Kohlenstoffatomen zu verstehen sind und die Phenylreste einfach, zweifach oder dreifach, insbesondere einfach oder zweifach in 4- und/oder 2-Stellung, mit Halogen, insbesondere Chlor oder Brom, substituiert sein können.

In der allgemeinen Formel III ist die alkalisch abspaltbare Gruppe beispielsweise ein Halogenatom, insbesondere ein Chlor- oder Bromatom, oder auch der Rest einer organischen Säure, z. B. der Toluolsulfonsäure.

Das Verfahren der vorstehend genannten Art zur Herstellung von Imidazolylvinylethern der allgemeinen Formel I ist aus der Druckschrift EP-A1-0 008 804 sowie mit geringen Abänderungen auch aus den Druckschriften DE-A1-2 757 113 und DE-A1-2 839 388 bekannt. Nach diesem bekannten Verfahren wird die Kondensation des als 1-Arylcarbonylalkylimidazol auffaßbaren Ketons der allgemeinen Formel II mit der eine in der Kondensation abspaltbare Gruppe Z enthaltenden Substanz der allgemeinen Formel III in Gegenwart einer alkalischen Base, insbesondere Natriumhydrid, in einem polaren aprotischen Lösungsmittel, insbesondere in Hexamethylphosporsäuretriamin oder in Dimethylformamid, durchgeführt. Neben dem genannten Natriumhydrid können als Kondensationsmittel auch Alkalimetalle, Erdalkalimetalle, deren Hydride und Alkoholate, lithiumorganische Verbindungen oder unsubstituiertes oder N-substituiertes Natriumamid eingesetzt werden.

Die zur Durchführung des bekannten Verfahrens erforderlichen Kondensationsmittel und aprotischen Lösungsmittel sind teure Substanzen. Außerdem sind sie in der Handhabung toxikologisch und ökologisch bedenklich. Schließlich führt die bekannte Kondensationsreaktion zu einem Isomerengemisch, in dem sowohl das E-Isomere als auch das Z-Isomere der Imidazolylvinylether der allgemeinen Formel I zu ungefähr gleichen Teilen vorliegt. Im Hinblick darauf, daß die E-Isomeren in fast allen Fällen hinsichtlich ihrer bakteriziden und fungiziden Eigenschaften die aktiveren Isomeren sind und das als Produkt erhaltene Isomerengemisch daher chromatographisch getrennt werden muß, ist das bekannte Verfahren auch vom wirtschaftlichen Standpunkt her für die Produktion größerer Wirkstoffmengen nur mäßig geeignet. Hinzu kommt, daß statt der mit der Kondensation angestrebten O-Alkylierung häufig in unerwünschter Weise auch eine C-Alkylierung eintritt, so daß das nach dem bekannten Verfahren erhaltene Reaktionsprodukt auch aus diesem Grund einer zusätzlichen säulenchromatographischen Trennung bedarf. Für die wirtschaftliche industrielle Produktion der Imidazolylvinylether der allgemeinen Formel I ist dies ungünstig.

Der Erfindung lag die Aufgabe zugrunde, das eingangs beschriebene bekannte Verfahren zur Herstellung von Imidazolylvinylethern der allgemeinen Formel I wirtschaftlicher zu gestalten, auf die Verwendung toxikologisch und ökologisch weniger bedenklicher Reaktionssysteme umzustellen und den Zugang zu den E-Isomeren zu erleichtern und wirtschaftlicher zu gestalten.

Zur Lösung dieser Aufgabe wurde das eingangs beschriebene bekannte Verfahren durch die erfindungsgemäßen Maßnahmen in kennzeichnender Weise dahingehend umgestaltet, daß die Umsetzung nicht in einem aprotischen Lösungsmittel, sondern in der wässrigen Lösung einer starken zu organischen Base durchgeführt wird, also in einem typisch protischen Reaktionssystem.

Die nach dem Verfahren der Erfindung zur Durchführung der Kondensationsreaktion benutzten Basen sind vor allem wesentlich preiswerter als die nach dem Stand der Technik erforderlichen polaren aprotischen Lösungsmittel und die erforderlichen aufwendigen Kondensationsmittel, beispielsweise das in einem Öl dispergierte Natriumhydrid, das nach dem Stande der Technik vorzugsweise verwendet wird. Darüber hinaus sind die im Verfahren der Erfindung verwendeten wässrigen anorganischen Basen auch toxikologisch und ökologisch wesentlich unbedenklicher und verfahrenstechnisch preiswerter zu handhaben als die bekannterweise benutzten Reaktionssysteme.

Von großem Vorteil ist schließlich, daß nach dem Verfahren der Erfindung die Imidazolylvinylether der allgemeinen Formel I bei Durchführung der Reaktion in einer wässrigen Lösung einer starken anorganischen Base in Form eines Isomerengemisches anfallen, das zum ganz überwiegenden Teil aus dem E-Isomeren besteht. Aus diesem Produktgemisch kann das fast reine E-Isomere durch einfache Kristallisation gewonnen werden. Eine chromatographische Trennung des Isomerengemisches kann in den allermeisten Fällen entfallen. Dabei reicht die durch einfache Kristallisation des E-Isomeren erzielbare stereochemische Reinheit bereits fast immer aus, um das Kristallisat direkt der therapeutischen Verwendung zuzuführen.

Als wässrige Lösung einer starken anorganischen Base wird vorzugsweise eine konzentrierte wässrige Natronlauge, insbesondere eine mindestens zweinormale wässrige Natronlauge, eingesetzt.

Nach einer Ausgestaltung der Erfindung wird die Kondensationsreaktion zwischen den Substanzen der allgemeinen Formeln II und III vorzugsweise in Gegenwart einer zweiten flüssigen, nämlich einer organischen Phase im Reaktionssystem durchgeführt. Zu diesem Zwecke wird dem wässrigen Reaktionssystem insbesondere ein flüsssiger apolarer Kohlenwasserstoff zugesetzt, vor allem Benzol oder Toluol. Zur Verbesserung des Phasenübergangs wird dabei vorzugsweise in Gegenwart eines Phasentransferkatalysators gearbeitet.

Bekannt für die Eignung als Phasentransferkatalysator sind quaternäre Ammonium- oder Phosphoniumsalze. Beispiele solcher Salze finden sich zusammengestellt in der Monographie von W. P. Weber und G. W. Gockel, Phase Transfer Catalysis in Organic Synthesis, Berlin, Springer Verlag, 1977. Als besonders geeignet für diesen Zweck erwies sich Tetrabutylammoniumhydroxid, ferner auch Triethylbenzylammoniumchlorid und Benzyl-dimethyl-tetradecylammoniumbromid.

Das Aufarbeiten und Abtrennen des Reaktionsproduktes erfolgt in an sich bekannter und gebräuchlicher Weise durch Destillation, Filtration oder Fällen, insbesondere durch Fällen mit Säuren, die mit den Imidazolylvinylethern der allgemeinen Formel I Säureadditionssalze bilden. Vorzugsweise erfolgt

die Fällung mit konzentrierter Salpetersäure.

Beim vorsichtigen Kristallisierenlassen der Produkte, insbesondere der Salze, können die sterisch fast reinen E-Isomeren der Imidazolylvinylether der allgemeinen Formel I erhalten werden.

Die Erfindung wird im folgenden an Hand von Ausführungsbeispielen näher erläutert.

### Beispiel 1

2-(2,4-Dichlorphenyl)-2-methoxy-1-(imidazol-1-yl)-ethylennitrat

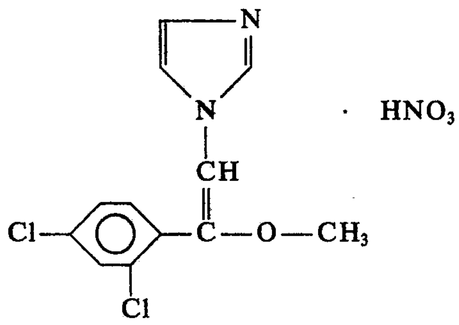

Ein Gemisch von 58,32 g (0,2 mol) 1-(2,4-Dichlorphenacyl)-imidazol und 200 ml 2n NaOH wird bei Raumtemperatur tropfenweise und unter Rühren mit 37,25 g (0,2 mol) p-Toluolsulfonsäuremethylester versetzt. Anschließend wird das Reaktionsgemisch 3 d bei Raumtemperatur gerührt. Danach wird das Reaktionsgemisch zunächst mit Ether und dann mit Chloroform ausgeschüttelt. Die Extrakte werden über Natriumsulfat getrocknet, anschließend vereinigt und eingedampft. Das als Rückstand verbleibende Öl wird in Dichlormethan gelöst und auf einer mit 500 g Kieselgel gefüllten Säule chromatographiert. Die Einheitlichkeit der Eluatfraktionen wird dünnschichtchromatographisch überprüft. Die einheitlichen Fraktionen werden vereinigt. Von den vereinigten Fraktionen wird das Lösungsmittel abgezogen. Der verbleibende ölige Rückstand wird in Ether gelöst. Aus dieser Lösung wird das Zielprodukt mit 65%iger Salpetersäure als Nitrat gefällt. Die Kristalle werden durch Filtration abgetrennt und aus Essigester umkristallisiert. Das gereinigte Endprodukt weist einen Schmelzpunkt von 152 bis 153°C auf. Die Einheitlichkeit, Reinheit und Identität des so erhaltenen Nitrats wird durch Elementaranalyse und durch das IR-Spektrum gesichert.

### Beispiel 2

2-(2,4-Dichlorphenyl)-2-(2-(4-chlorphenoxy)-ethoxy)-1-(imidazol-1-yl)-ethylennitrat

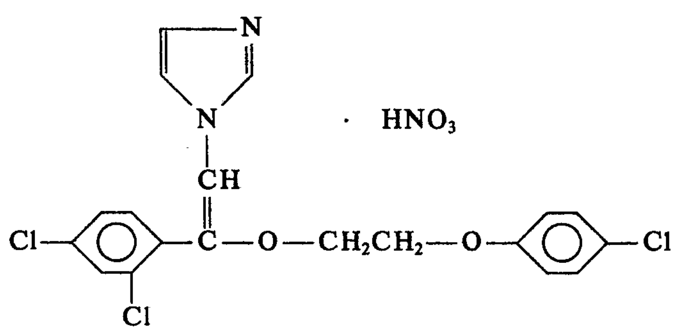

Ein Gemisch von 291,6 g (1 mol) 1-(2,4-Dichlorphenacyl)-imidazol, 800 ml einer 50%igen wässrigen Natronlauge, 80 ml einer 40%igen wässrigen Tetrabutylammoniumhydroxidlösung und 800 ml Toluol wird auf 50°C erwärmt. Bei dieser Temperatur werden im Verlauf von 3 h 353,3 g (1,5 mol) 1-Brom-2-(4-chlorphenoxy)-ethan, gelöst in 200 ml Toluol, unter kräftigem Rühren eingetropft. Anschließend wird weitere 1,5 h bei 50°C gerührt. Nach dem Abkühlen werden anschließend die beiden flüssigen Phasen getrennt. Die organische Phase wird zweimal mit Wasser gewaschen, über Natriumsulfat getrocknet und dann unter vermindertem Druck eingedampft. Als Rückstand verbleibt ein dunkles Öl, das in 850 ml Essigester gelöst wird. Die Lösung wird dann mit 100 ml einer 65%igen wässrigen Salpetersäure versetzt. Die salpetersaure Lösung wird dann abgekühlt und portionsweise mit insgesamt 500 ml Ether versetzt. Dabei fällt das Produktnitrat aus. Der Niederschlag wird auf einem Saugfilter abgetrennt und anschließend mit Essigester und Ether gewaschen. Die noch schwach gelblich gefärbten Kristalle werden aus Essigester umkristallisiert. Das gereinigte und praktisch farblose End

produkt, das in einer Ausbeute von 42% der theoretischen Ausbeute erhalten wird, hat einen Schmelzpunkt von 145 bis 147°C. Die Reinheit und Identität des erhaltenen Produktnitrats wird durch Elementaranalyse und IR-Spektrum gesichert.

Beispiel 3

E-1-(2,4-Dichlorphenyl)-1-(2-(4-chlorphenoxy)-ethoxy)-2-(imidazol-1-yl)-propennitrat

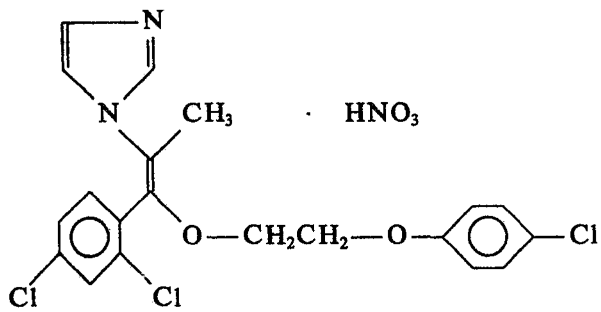

166 g (0,5 mol) 1-(2,4-Dichlorphenyl)-2-(imidazol-1-yl)-propan-1-on, 400 ml einer 50%igen wässrigen Natronlauge, 400 ml Benzol und 40 ml einer 40%igen wässrigen Lösung von Tetrabutylammoniumhydroxid werden vermischt und unter kräftigem Rühren auf 50°C erwärmt. In die gerührte und erwärmte Lösung werden im Verlauf von 10 h 141 g 1-Brom-2-(4-chlorphenoxy)-ethan, gelöst in 400 ml Benzol, eingetropft. Das Gemisch wird anschließend weitere 15 h bei 50°C gerührt. Dann wird unter analytischer Überwachung, hier mit Dünnschichtchromatographie, solange bei Raumtemperatur weitergerührt, in diesem Fall weitere 24 h, bis die Reaktion abgeschlossen ist. Nach Abschluß der Reaktion wird das Reaktionsgemisch mit soviel Wasser und Chloroform versetzt, daß die wässrige Phase leichter als die organische Phase wird. Anschließend werden die organischen und die wässrige Phase getrennt. Die organische Phase wird über Natriumsulfat getrocknet. Die Lösungsmittel werden unter vermindertem Druck abgezogen. Als Rückstand verbleibt ein dunkles Öl, das in ca. 500 ml Essigester gelöst wird. Die Lösung wird mit 250 ml Ether verdünnt und dann mit 65%iger wässriger Salpetersäure auf einen pH-Wert von 5 eingestellt. Die so salpetersauer gestellte Lösung wird dann im Kühlschrank abgekühlt. Das dabei ausfallende Rohprodukt wird anschließend aus einem Gemisch aus 250 ml Essigester und 250 ml Ethanol umkristallisiert. Das gereinigte Produkt weist einen Schmelzpunkt von 121°C auf und wird analytisch als nahezu reines E-Isomer des Propennitrats identifiziert.

Beispiel 4

2-(2,4-Dichlorphenyl)-2-butoxyethoxy-1-(imidazol-1-yl)-ethylennitrat

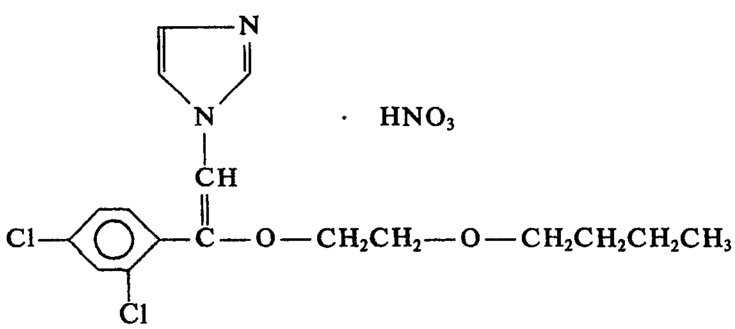

Das im Beispiel 2 beschriebene Verfahren wird mit der Abänderung wiederholt, daß statt des 1-Brom-2-(4-chlorphenoxy)-ethans die äquimolare Menge 1-Brom-2-n-butoxy-ethan eingesetzt wird. Das gereinigte Nitrat weist nach dem Umkristallisieren einen Schmelzpunkt von 191 bis 192°C auf.

Beispiel 5

1-(2,4-Dichlorphenyl)-1-(2,4-dichlorphenoxyethoxy)-2-(imidazol-1-yl)-propennitrat

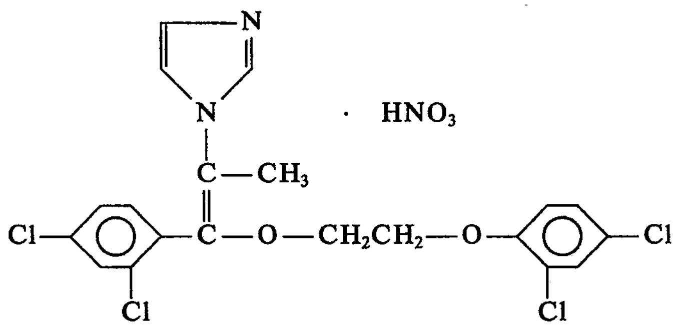

Das im Beispiel 3 beschriebene Verfahren wird mit der Abänderung wiederholt, daß statt des 1-Brom-2-(4-chlorphenoxy)-ethans die äquimolare Menge 1-Brom-2-(2,4-dichlorphenoxy)-ethan eingesetzt wird.

Das gereinigte Endprodukt, das fast ausschließlich aus dem E-Isomer besteht, weist einen Schmelzpunkt von 118 bis 120° C auf.

Beispiel 6

2-(2,4-Dichlorphenyl)-2-(4-chlorbenzyloxy)-1-(imidazol-1-yl)-ethylennitrat

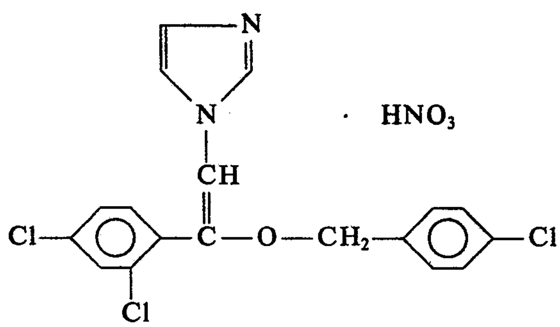

Das im Beispiel 2 beschriebene Verfahren wird mit der Abänderung wiederholt, daß statt des 1-Brom-2-(4-phenoxy)-ethans die äquimolare Menge p-Brommethylchlorbenzol eingesetzt wird.

Das umkristallisierte Endprodukt weist einen Schmelzpunkt von 117 bis 118° C auf.

Beispiel 7

2-(2,4-Dichlorphenyl)-2-(2-(4-chlorbenzyloxy)-ethoxy)-1-(imidazol-1-yl)-ethylennitrat

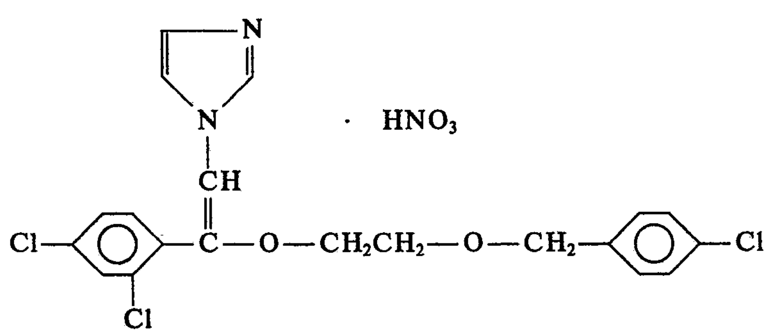

Das im Beispiel 2 beschriebene Verfahren wird mit der Abänderung wiederholt, daß statt des 1-Brom-2-(4 phenoxy)-ethans die äquimolare Menge 1-Brom 2-(4-chlorbenzyloxy)-ethan eingesetzt wird.

Das umkristallisierte Endprodukt, das eine auffallend hohe biozide Aktivität aufweist, hat einen Schmelzpunkt von 119 bis 121° C.

**Patentansprüche**

1. Verfahren zur Herstellung von Imidazolylvinylethern der allgemeinen Formel I

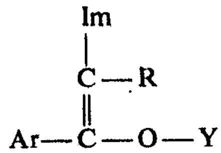

(I)

und deren Säureadditionssalze, wobei

Ar Phenyl, Naphthyl, Thienyl oder Pyridyl bedeutet, wobei diese Ringe einfach oder mehrfach substituiert sein können und die Substituenten unabhängig voneinander Halogen, niederes Alkyl oder Cycloalkyl, mit bis zu 6 Kohlenstoffatomen, Trifluormethyl, niederes Alkoxy und niederes Alkylthio mit jeweils 1 bis 6 Kohlenstoffatomen im Alkylteil, Phenyl, Benzyl, Cyano, Nitro oder Amino sein können,

R Wasserstoff, ein unverzweigtes oder verzweigtes Alkyl mit bis zu 6 Kohlenstoffatomen, Phenyl oder ein phenyl-substituiertes Alkyl mit bis zu 6 Kohlenstoffatomen im Alkylteil sein kann, wobei das Phenyl jeweils mit Halogen oder Alkyl oder Alkoxy mit bis zu 6 Kohlenstoffatomen im Alkylteil substituiert sein kann,

Im die 1-H-Imidazol-1-yl-gruppe bedeutet, die einfach mit der Nitrogruppe oder einfach, zweifach oder dreifach mit Halogen oder einem Alkyl- oder Alkoxyrest mit 1 bis 4 Alkylkohlenstoffatomen substituiert sein kann, und

Y ein unverzweigtes, verzweigtes oder cyclisches, gesättigtes oder ungesättigtes, eine Doppelbindung aufweisendes Alkyl mit bis zu 6 Kohlenstoffatomen, Phenyl, Naphthyl, Thienyl oder Pyridyl oder ein mit Phenyl, Naphthyl, Pyridyl oder Thienyl substituiertes Alkyl mit 1 bis 6 Kohlenstoffatomen im Alkylteil bedeutet, wobei alle vorstehend genannten Reste, und zwar auch die Alkylreste, jeweils in der vorstehend für den Rest Ar genannten Weise substituiert sein können und eine direkt an den Ethersauerstoff gebundene Alkylgruppe oder eine direkt an den Ethersauerstoff gebundene Alkylengruppe einmal oder zweimal in der Kette die zweiwertigen Gruppen $-O-$, $-S-$, $-SO-$ oder $-SO_2-$ aufweisen kann oder eine dieser Gruppen in $\omega$-Stellung zum Ethersauerstoff endständig tragen kann, wobei diese Gruppe dann mit einem der für Ar oben genannten Reste abgesättigt ist,

durch Umsetzen eines entsprechenden Ketons der allgemeinen Formel II

$$Ar-CO-CHR-Im \qquad (II)$$

in der Ar, R und Im die vorstehend genannte Bedeutung haben, in einem alkalischen Medium mit einer Verbindung der allgemeinen Formel III

$$Z-Y \qquad (III)$$

in der Z eine alkalisch abspaltbare Gruppe ist, und Abtrennen des Reaktionsproduktes, dadurch gekennzeichnet, daß die Umsetzung in der wässrigen Lösung einer starken anorganischen Base durchgeführt wird.

2. Verfahren nach Anspruch 1, dadurch gekennzeichnet, daß die Umsetzung in Gegenwart einer zweiten flüssigen organischen Phase durchgeführt wird.

3. Verfahren nach Anspruch 2, dadurch gekennzeichnet, daß bei Durchführung der Umsetzung im zweiphasigen System in Gegenwart eines Phasentransferkatalysators gearbeitet wird.

4. Verfahren nach einem der Ansprüche 1 bis 3, dadurch gekennzeichnet, daß als wässrige Lösung einer starken anorganischen Base konzentrierte Natronlauge dient.

5. Verfahren nach einem der Ansprüche 2 bis 4, dadurch gekennzeichnet, daß Benzol oder Toluol als zweite Phase eingesetzt wird.

6. Verfahren nach einem der Ansprüche 2 bis 5, dadurch gekennzeichnet, daß der Phasentransferkatalysator Tetrabutylammoniumhydroxid ist.

7. Verfahren nach einem der Ansprüche 1 bis 6 zur Herstellung der E-Isomeren der allgemeinen Formel I durch Kristallisieren des so erhaltenen Reaktionsproduktes.

## Claims

1. A method for producing imidazolyl vinyl ethers having the general formula I

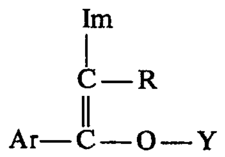

(I)

and the acid addition salts thereof, wherein

Ar is phenyl, naphthyl, thienyl or pyridyl which rings can be mono or multiple substituted, and that substituents independently from each other can be halogen, lower alkyl or cycloalkyl having up to 6 carbon atoms, trifluoromethyl, lower alkoxy and lower alkylthio each having 1 to 6 carbon atoms in the alkyl moiety thereof, phenyl, benzyl, cyano, nitro or amino,

R is hydrogen, a linear or branched alkyl having up to 6 carbon atoms, phenyl or a phenyl substituted alkyl having up to 6 carbon atoms in the alkyl moiety, whereat said phenyl in each case can be substituted with halogen or alkyl or alkoxy each of which has up to 6 carbon atoms in its alkyl moiety,

Im is the 1-H-imidazol-1-yl group which can be mono substituted with the nitro group or can be mono, double or triple substituted with halogen or an alkyl or alkoxy group having 1 to 4 carbon atoms in the alkyl moiety and

Y is a linear, branched or cyclic saturated or ethylenically unsaturated alkyl having up to 6 carbon atoms, phenyl, naphthyl, thienyl or pyridyl or is an alkyl having 1 to 6 carbon atoms, which is substituted with phenyl, naphthyl, pyridyl or thienyl, whereat all aforementioned groups including the alkyl groups each can be substituted as defined above for the group Ar and whereat an alkyl group directly bound to the ether oxygen or an alkylene group directly bound to the ether oxygen once or twice in its molecular chain can have incorporated the divalent groups —O—, —S—, —SO— or —$SO_2$— or can carry one of those groups in a terminal position in $\omega$-position relative to the ether oxygen, in which latter case said group then is saturated with one of the groups as mentioned above for Ar,

by reacting a corresponding ketone of the general formula II

Ar—CO—CHR—Im (II)

in which Ar, R and Im have the same meaning as defined above, in an alkaline medium with a compound having the general formula III

Z—Y (III)

in which Z is a group which can be split off in an alkaline medium, and recovering the reaction product, characterized in that said reaction is carried out in aqueous solution of a strong inorganic base.

2. A method according to claim 1, characterized in that said reaction is carried out in the presence of a second liquid organic phase.

3. A method according to claim 2, characterized in that the reaction is carried out in the presence of a phase transfer catalyst when working in a two-phase system.

4. A method according to one of the claims 1 to 3, characterized in that said aqueous solution of a strong inorganic phase is a concentrated sodium hydroxide solution.

5. A method according to one of the claims 2 through 4, characterized in that, benzene or toluene are used as said second phase.

6. A method according to one of the claims 2 through 5, characterized in that, said phase transfer catalyst is tetrabutyl ammonium hydroxide.

7. A method according to one of the claims 1 through 6 for producing the E-isomer of the substance according to the general formula I by crystallization of the thus obtained reaction product.

**Revendications**

1. Procédé de préparation d'éthers imidazolylvinyliques (et de leurs sels d'addition d'acides) répondant à la formule générale I:

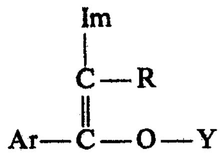

(I)

dans laquelle:

Ar représente un phényle, un naphtyle, un thiényle ou un pyridyle, chacun de ces noyaux pouvant porter un substituant ou plusieurs substituants qui peuvent être chacun, indépendamment les uns des autres, un halogène, un radical allyle ou cycloalkyle inférieur contenant au plus 6 atomes de carbone, un trifluorométhyle, un alcoxy inférieur ou un alkylthio inférieur contenant chacun de 1 à 6 atomes de carbone dans la partie alkylique, un phényle, un benzyle, un cyano, un nitro ou un amino,

R représente l'hydrogène, un alkyle contenant au plus 6 atomes de carbone, linéaire ou ramifié, un phényle ou un phénylalkyle dont l'alkyle contient au plus 6 atomes de carbone et dont le phényle peut porter un halogène ou un radical alkyle ou alcoxy contenant au plus 6 atomes de carbone dans la partie alkylique,

Im représente un radical 1-H-imidazolyle-1 qui peut porter un radical nitro, auquel car il est monosubstitué, ou un, deux ou trois substituants pris dans l'ensemble constitué par les halogènes ainsi que les alkyles et les alcoxy contenant de 1 à 4 atomes de carbone dans leurs parties alkyliques, et

Y représente un alkyle contenant au plus 6 atomes de carbone, ramifié, non ramifié ou cyclique, saturé ou insaturé (avec une double liaison), un phényle, un naphtyle, un thiényle ou un pyridyle ou encore un alkyle contenant de 1 à 6 atomes de carbone et porteur d'un phényle, d'un naphtyle, d'un pyridyle ou d'un thiényle, tous les radicaux qui viennent d'être cités, y compris les radicaux alkyles, pouvant chacun être substitué de la manière indiquée cidessus pour Ar et un radical alkyle directement relié à l' atome d'oxygène de fonction éther ou un radical alkylène directement relié à l'atome d'oxygène de fonction éther pouvant renfermer une ou deux fois dans sa chaine un radical bivalent $-O-$, $-S-$, $-SO-$ ou $-SO_2-$ ou porter l'un de ces radicaux à l'extrémité de la chaine en position $\omega$ par rapport à l'atome d'oxygène de fonction éther, la liaison restante de ce radical pouvant alors être saturée par l'un des radicaux qui ont été cités plus haut pour Ar,

par réaction d'une cétone idoine répondant à la formule générale II:

$$Ar-CO-CHR-Im \qquad (II)$$

dans laquelle Ar, R et Im ont les significationsprécédemment données, dans un milieu alcalin, avec un composé répondant à la formule générale III

$$Z-Y \qquad (III)$$

dans laquelle Z représente un radical éliminable en milieu alcalin, et séparation du produit réactionnel, procédé caractérisé en ce qu'on effectue la réaction dans la solution aqueuse d'une base minérale forte.

2. Procédé selon la revendication 1 caractérisé en ce qu'on effectue la réaction en présence d'une deuxième phase organique liquide.

3. Procédé selon la revendication 2 caractérisé en ce que la réaction dans un système à deux phases est effectuée en présence d'un catalyseur de transfert de phase.

4. Procédé selon d'une quelconque des revendications 1 à 3, caractérisé en ce qu'on utilise, comme solution aqueuse d'une base minérale forte, une solution concentrée d'hydroxyde de sodium.

5. Procédé selon l'une quelconque des revendications 2 à 4, caractérisé en ce qu'on utilise le benzène ou le toluène comme deuxième phase.

6. Procédé selon l'une quelconque des revendications 2 à 5, caractérisé en ce que le catalyseur de transfert de phase est l'hydroxyde de tétrabutylammonium.

7. Procédé selon l'une quelconque des revendications 1 à 6 pour la préparation des isomères E de formule générale I par cristallisation du produit réactionnel obtenu.